# EUROPÄISCHE PATENTSCHRIFT

(11) **EP 2 825 028 B1**
(45) Veröffentlichungstag und Bekanntmachung des Hinweises auf die Patenterteilung: **08.08.2018**
(21) Anmeldenummer: 13709467.8
(22) Anmeldetag: 18.03.2013
(51) Int. Cl.: A01J 5/01, A01J 5/013, G01F 1/66

(54) **VERFAHREN ZUR BESTIMMUNG DER QUALITÄT UND/ODER ZUSAMMENSETZUNG VON MILCH, INSBESONDERE WÄHREND EINES MELKVORGANGS**
METHOD FOR DETERMINING THE QUALITY AND/OR COMPOSITION OF MILK, IN PARTICULAR DURING A MILKING PROCESS
PROCÉDÉ DE DÉTERMINATION DE LA QUALITÉ ET/OU DE LA COMPOSITION DU LAIT, EN PARTICULIER PENDANT UNE PROCÉDURE DE TRAITE

(30) Priorität: 16.03.2012 DE 102012005205
(43) Veröffentlichungstag der Anmeldung: 21.01.2015
(73) Patentinhaber: GEA Farm Technologies GmbH, 59199 Bönen (DE)
(72) Erfinder: SCHÖNROCK, Karsten, 58313 Herdecke (DE); KRASUTZKI, Marek, 48291 Telgte (DE); BIELETZKI, Sascha, 59199 Bönen (DE); BALKENHOL, Reinhard, 33098 Paderborn (DE)
(74) Vertreter: KNH Patentanwälte Neumann Heine Taruttis PartG mbB
(86) Internationale Anmeldenummer: PCT/EP2013/055521
(87) Internationale Veröffentlichungsnummer: WO 2013/135903

(56) Entgegenhaltungen:
- EP-A1- 2 036 470
- WO-A1-01/19170
- WO-A1-01/56369
- WO-A1-2005/093387
- GB-A- 2 352 033
- US-A- 5 116 119

## Beschreibung

Die Erfindung betrifft ein Verfahren zur Qualitätsbestimmung von Milch.
Rohmilch ist ein bedeutsames Lebensmittel und ein wichtiger Rohstoff für die Nahrungsmittelindustrie. Zum Schutz des Verbrauchers, zur technischen Verarbeitungsfähigkeit sowie zur Marktlenkung muss Rohmilch bestimmten nationalen sowie internationalen Qualitätsanforderungen entsprechen. Beispielhaft wird hierzu auf die Verordnung über Anforderungen an die Hygiene beim Herstellen, Behandeln und Inverkehrbringen bestimmter Lebensmittel tierischen Ursprungs (Tierische Lebensmittel - Hygieneverordnung - Tier-LMHV) oder die Verordnung (EG) Nr. 852/2004 des Europäischen Parlaments und des Rates vom 29. April 2004 über Lebensmittelhygiene verwiesen.
Bei Melkvorrichtungen und -verfahren generell und insbesondere beim automatischen und automatisierten Melken mit halb- und auch vollautomatischen Melksystemen spielen erweiterte Funktionen eine zunehmend größere Rolle. Insbesondere steht die Gewährleistung von Qualitätsstandards der Milch, insbesondere auch die Prüfung auf sinnfällig veränderte Milch im Vordergrund. Sinnfällig veränderte Milch bedeutet, dass die Milch durch Blut, Eiter oder durch Flocken verunreinigt ist, das heißt in der Erscheinung hinsichtlich Farbe, Geruch oder Konsistenz unerwünscht auffällig verändert ist. Zur Bestimmung der Sinnfälligkeit sind verschiedene Verfahren bekannt. Einige Verfahren nutzen Leitwert- oder Dichtemessungen oder Siebe, um den Gehalt an Flocken in der Milch zu bestimmen. Andere erfassen sinnfällige Veränderungen hinsichtlich farblicher Veränderungen der Milch.
Durch die EP 1 287 737 A2 ist ein Verfahren zur qualitativen Charakterisierung von Milch während eines Melkvorgangs bekannt. Hierzu wird wenigstens ein akustisches Sendesignal ausgestrahlt, das durch die Milch beeinflusst wird. Ein Empfangssignal wird aufgenommen. Aus dem aufgenommenen Empfangssignal wird wenigstens ein Kennwert abgeleitet, der geeignet ist, die Qualität der Milch zu charakterisieren. Durch dieses Verfahren kann die Qualität der Milch bereits beim Melken bestimmt werden. Dadurch wird erreicht, dass z. B. nur jene Milch in einen Sammelbehälter geleitet wird, die qualitativ zur Weiterverarbeitung geeignet ist. Dies reduziert die Gefahr der Verunreinigung der Milch im Sammelbehälter durch Milch einer ungenügenden Qualitätsstufe.

Durch die WO 2002/084260 ist ein Verfahren zur optischen Charakterisierung von Milch mit einem optischen System bekannt. Zunächst wird das optische System in mindestens einer Frequenz kalibriert. Anschließend wird mindestens ein optisches Spektrum der Milch mindestens einer Frequenz aufgenommen, woraufhin das erhaltene Spektrum mit Hilfe einer ersten Abbildung in einem Farbraum mindestens einen Farbvektor abgebildet wird. Danach wird mindestens ein Farbvektor mit Hilfe einer zweiten Abbildung einem Merkmalsraum auf einen Merkmalsvektor abgebildet. Durch dieses Verfahren wird die Milch berührungslos, in Echtzeit und im Durchflussverfahren charakterisiert. Durch die Kalibrierung wird die optische Übertragungsfunktion des optischen Systems festgestellt, damit diese später aus den mit Milch aufgenommenen optischen Spektren herausgerechnet werden kann. Bspw. erfolgt für die Rohwerte des Sensors der Offset-Abgleich sowie eine automatische Kalibrierung mit Referenzwerten. Nach diesem Schritt werden die einzelnen Rohwerte noch für sich isoliert betrachtet.

Auch die EP 1 000 535 befasst sich mit der Ermittlung der Qualität von Milch. Hierzu wird ein Verfahren vorgeschlagen, mit dem eine Quelle die Milch nacheinander mit Strahlung unterschiedlicher Wellenlänge bestrahlt. Ein Empfänger misst während zumindest eines Teils der Zeit, in der die Quelle eingeschaltet ist, die Stärke der reflektierten Strahlung über einen Zeitraum hinweg. Die Werte der auf diese Weise gemessenen Strahlungsstärke werden in einem Speicher gespeichert. Die Werte werden miteinander sowie mit früheren Werten verglichen, die während einer früheren Messung aufgezeichnet wurden. Das Ergebnis dieses Vergleichsvorgangs wird angezeigt. Bei einem solchen Verfahren besteht das Problem, dass die Werte der Strahlungsstärke der Milch stark von der Menge an Umgebungslicht variieren. Es wird daher vorgeschlagen, dass während einer Messung die Quelle abgeschaltet wird, weil der Empfänger während zumindest eines Teils der Zeit, in der die Quelle abgeschaltet ist, die Stärke der reflektierten Strahlung über einen Zeitraum hinweg misst. Die Werte der auf diese Weise gemessenen Strahlungswerten werden als Hintergrundwerte in einem Speicher gespeichert. Die Hintergrundwerte werden in die Werte integriert, die während der Zeit, als die Quelle eingeschaltet war, gewonnen wurden. Die durch die Hintergrundwerte berichtigten Werte werden in einem Speicher gespeichert. Durch diese Vorgehensweise wird eine Korrektur der Strahlungsstärke, die gemessen wird, und die in Quellen eingeschaltet sind, ermöglicht.
Durch die WO 01/056369 ist eine Vorrichtung zum Ermitteln von physischen Anomalien in Milch bekannt. Die Vorrichtung weist mindestens eine Lichtquelle auf, um Milch und/oder Milchproben mit Licht von roter und/oder grüner und/oder blauer Farbe zu bestrahlen. Es ist mindestens ein Lichtsensor zum Messen der Stärke von Licht, das von der Milch reflektiert und/oder zerstreut und/oder durchgelassen wird, vorgesehen. Die Messdaten werden mittels eines Computers verarbeitet. Der Computer ist dazu derart programmiert, dass auf der Basis eines Vergleichs der von den gemessenen Lichtstärken abgeleiteten Werte mit Referenzwerten oder durch mehrere Diagnosen für die Anomalien in der Milch aus einer Vielzahl von gespeicherten Diagnosemöglichkeiten auszuwählen. Weitere Verfahren und Vorrichtungen sind aus der WO 2005/093387 A1 bekannt. Hiervon ausgehend liegt der vorliegenden Erfindung die Zielsetzung zugrunde, ein verbessertes Verfahren zur Bestimmung der Qualität der Milch anzugeben.
Diese Aufgabe wird gelöst durch ein Verfahren zur Ermittlung der Qualität und/oder der Zusammensetzung von Milch gemäß den Merkmalen des Patentanspruchs 1. Weitere vorteilhafte Ausgestaltungen der Erfindung sind in den abhängig formulierten Patentansprüchen angegeben. Die in den Patentansprüchen einzeln aufgeführten Merkmale sind in beliebiger, technologisch sinnvoller, Weise miteinander kombinierbar und können durch erläuternde Sachverhalte aus der Beschreibung ergänzt werden, wobei weitere Ausführungsvarianten der Erfindung aufgezeigt werden.
Nach dem erfindungsgemäßen Verfahren zur Ermittlung der Qualität und/oder der Zusammensetzung von Milch, während eines Melkvorgangs wird vorgeschlagen, dass der Füllstand der Milch in einer Kammer bestimmt wird. Die Kammer kann durch einen Abschnitt eines Milchschlauchs oder dergleichen gebildet werden. Erfindungsgemäß durchströmt die Milch die Kammer. Zum Zeitpunkt der Messung bzw. Bestimmung der Qualität der Milch wird der Füllstand, das heißt der Flüssigkeitsspiegel der Milch in der Kammer bestimmt. Alternativ oder zusätzlich kann die Kammer innerhalb einer Leitung ausgebildet werden. Die Kammer kann ein vorgegebenes Volumen aufweisen, in welches die Milch während der Durchströmung der Leitung einströmt. Es ist auch möglich, dass die Kammer an einer Bypassleitung angeordnet wird. Ggf. kann eine Probe der Milch gezogen werden und diese in einer Kammer untersucht werden. Durch das erfindungsgemäße Verfahren kann eine Flockendetektion, eine Harnstoff-, Laktose- und/oder eine Fettbestimmung in der Milch erreicht werden. Ferner kann mittels des Verfahrens auch eine Bestimmung der somatischen Zellen in der Milch durchgeführt werden.

Vorteilhaft ist es jedoch, wenn während des Melkvorgangs die Kammer durch den Abschnitt eines Leitungssystems des Melksystems gebildet wird, so dass bereits während des Melkvorgangs die Qualität und/oder der Zusammensetzung der Milch ermittelt wird.

Nachdem der Füllstand der Milch in einer Kammer bestimmt wurde, wird die Milch mit wenigstens einer Strahlung einer vorgegebenen Wellenlänge bestrahlt. Die Stärke der reflektierten Strahlung wird gemessen. Der Füllstand sowie die Stärke der reflektierten Strahlung bilden ein Wertepaar. In einem Speicher sind Kennwerte gespeichert. Dem ermittelten Wertepaar wird ein Kennwert zugeordnet. Aus dem so ermittelten Kennwert kann eine Aussage über die Qualität und/oder die Zusammensetzung der Milch getroffen werden.

Bevorzugt ist ein Verfahren, bei dem der Füllstand der Milch in der Kammer berührungslos gemessen wird. Besonders bevorzugt ist dabei eine Ausgestaltung des Verfahrens, bei dem die Milch mit rotem Licht bestrahlt und aus der Stärke des reflektierten roten Lichts der Füllstand der Milch in der Kammer bestimmt wird.

Nach einer weiteren vorteilhaften Ausgestaltung des Verfahrens wird vorgeschlagen, dass die vorgegebene Wellenlänge des Lichts der Wellenlänge des grünen oder blauen Lichts entspricht. Aus der Stärke des reflektierten grünen Lichts kann eine Aussage darüber getroffen werden, ob und in welcher Konzentration sich Hämoglobin in der Milch befindet.

Die Stärke der reflektierten Strahlung des blauen Lichts kann dazu verwendet werden, Informationen darüber zu erhalten, wie hoch der wässrige Anteil in der Milch ist. Der wässrige Anteil der Milch kann ein bedeutsames Indiz sein, dass eine E.coli-Infektion vorliegt.

Die Stärke des reflektierten blauen Lichts kann auch als Überwachungssignal für eine durchzuführende Reinigung der Kammer verwendet werden. Die Kammer wird mittels Wasser oder einer Wasser enthaltenden Flüssigkeit enthaltend Wasser gereinigt, so kann aus der Stärke der reflektierten Teile des blauen Lichts eine Aussage darüber getroffen werden, ob der Reinigungsvorgang ordnungsgemäß abläuft. Da der Reinigungsvorgang bzw. die Kammer ein Teil eines Leitungssystems der Melkanlage darstellen kann, kann auch eine Überwachung der Reinigung des Leitungssystems des Melksystems erzielt werden.

Die sich in der Kammer befindende Milch kann mit weißem Licht für eine vorgegebene Zeitdauer bestrahlt werden. Ein entsprechend ausgebildeter Empfänger, der beispielsweise wenigstens einen optischen Filter aufweist empfängt das reflektierte Licht und liefert entsprechende Signale an eine Steuerung. Es können Empfänger und/oder Filter vorgesehen sein, die selektiv arbeiten, so dass lediglich reflektiertes Licht vorgegebener Wellenlängen ausgewertet wird bzw. werden. Der Empfänger oder die Empfänger können nacheinander oder gleichzeitig die zu den gewählten Wellenlängen des Lichts korrespondierende Messsignale an eine Steuerung weiterleiten. Nach einer vorteilhaften Ausgestaltung des Verfahrens wird vorgeschlagen, dass die Milch mit pulsierender Strahlung bestrahlt wird. Hierzu kann das Zeitmultiplex-Verfahren zum Einsatz kommen. Bei der Strahlung kann es sich um weißes Licht handeln. Bevorzugt wird die Milch nacheinander mit einer oder unterschiedlichen Wellenlängen des Lichts bestrahlt. Hierdurch können diskrete Antworten des Sensors über die Stärke des reflektierten Lichts in bestimmter Wellenlänge erhalten werden.

Die Kennwerte können in einem Kennlinienfeld oder auch als Look-up-Tabelle in einem Speicher gespeichert sein. Es ist auch möglich, eine mathematische Beziehung vorzusehen, mittels derer anhand der ermittelten Stärke der Reflektion des Lichts und der Höhe des Füllstands in der Kammer eine Information erhalten wird über den Anteil einer bestimmten Komponente in der Milch. Erfindungsgemäß wird zur Steigerung der Effizienz des Verfahrens zur Ermittlung der Qualität und/oder der Zusammensetzung der Milch vorgeschlagen, dass ein ein Mindestmilchstrom, der durch die Kammer oder in die Kammer fließt, detektiert wird. Danach erfolgt erst die Bestimmung des Füllstands in der Kammer. Die Detektion des Milchstroms kann bspw. mittels eines Leitwertsensors erfolgen. Der Leitwertsensor kann bspw. stromabwärts der Kammer angeordnet sein. Hierdurch ist auch sichergestellt, dass wenn der Leitwertsensor das Vorhandensein der Milch detektiert, diese auch durch die Kammer zumindest teilweise durchströmt ist. Statt eines Leitwertsensors können auch andere Sensoren verwendet werden, die die Strömung der Milch detektieren.
Nach dem erfindungsgemäßen Verfahren wird die Milch in der Kammer mit wenigstens einer Strahlung einer vorgegebenen Wellenlänge bestrahlt. Bevorzugt ist dabei ein Verfahren, bei dem die Strahlung eine monochromomatische Strahlung ist. Hierdurch wird auch eine höhere Sicherheit hinsichtlich der möglichen Aussagen über die Zusammensetzung und/oder die Qualität der Milch erreicht, da monochromatische Strahlung eine klar definierte Wellenlänge hat.

Alternativ oder zusätzlich zur Bestimmung des Füllstands mittels roten Lichts kann der Füllstand auch kapazitiv oder induktiv bestimmt werden.

Zur Verringerung des Auswerteaufwands kann gemäß einem weiteren vorteilhaften Gedanken vorgeschlagen werden, dass der Füllstand in der Kammer vorgegeben ist. Es wird festgestellt, ob der Füllstand, der vorgegeben wurde, erreicht ist. Dies kann kapazitiv oder induktiv erfolgen. Es können auch Widerstandsmessungen gemacht werden. Bspw. kann im oberen Bereich der Kammer ein Elektrodenpaar angeordnet werden, welches von der Milch benetzt wird. Liegt eine solche Benetzung vor, so kann ein Stromkreis geschlossen werden, was ein Indiz für das Erreichen eines vorgegebenen Füllstands. Aus der Kenntnis des Füllstands und der Stärke der reflektierten Strahlung kann dann auch bspw. aus einem Kennlinienfeld die Information über den Anteil oder die Konzentration einer Komponente in der Milch abgeleitet werden.

Zur Durchführung des Verfahrens wird vorgeschlagen, dass wenigstens ein Sensor mit wenigstens einer Lichtquelle und wenigstens ein Empfänger vorgesehen ist, wobei der Sensor auf ein herdenspezifisches oder tierindividuelles Weiß der Milch kalibriert wird. Das "Weiß" der Milch ist abhängig vom Fettgehalt in der Milch. Der Fettgehalt in der Milch ist tierindividuell. Ist ein Tieridentifikationssystem vorgesehen, so kann die Information über das tierindividuelle Weiß der Milch in einem Herdenmanagementsystem hinterlegt werden. Wird das Tier gemolken, so wird es zunächst identifiziert. Aus der Identifikation des Tiers folgen dann Daten, die dem System zur Verfügung gestellt werden. Anhand des tierindividuellen Weiß kann auch eine Veränderung der Milch oder deren Zusammensetzung abgeleitet werden. Jedenfalls kann das tierindividuelle Weiß der Milch gespeichert werden und für die Kalibrierung des Systems für einen nachfolgenden Melkvorgang dieses Tiers genutzt werden. Statt der Verwendung des tierindividuellen Weiß von Milch kann auch ein herdenspezifisches Weiß der Milch bereitgestellt werden. Das herdenspezifische Weiß der Milch folgt aus den Werten der einzelnen tierindividuellen Farbe der Milch.

Aus den ermittelten Kennwerten, oder über zumindest einen Kennwert können Schlussfolgerungen für nachfolgende Aktionen bzw. Behandlungen des Tiers abgeleitet werden. Wird bspw. festgestellt, dass der Kennwert einen vorgegebenen Grenzwert überschreitet, kann bspw. die Milch in einen Tank für nicht verwertbare Milch abgeleitet werden, um sicherzustellen, dass bspw. Milch mit einem hohen Blutgehalt nicht in einen Tank gelangt, der verwertbare Milch enthält. Aus den Kennwerten der Tiere kann auch auf den Gesundheitszustand der Herde geschlossen werden. Sind signifikante Kennwerte in bestimmten Bereichen vorhanden, so kann dies ein Indiz für Maßnahmen sein, um die Gesundheit der Tiere und somit auch der gesamten Herde abzuleiten bzw. durchzuführen.

Die Messung der Milch kann bei Kühen euterviertelindividuell erfolgen. Dies ist nicht zwingend notwendig, jedoch vorteilhaft.

Soweit die vorstehenden Ausführungen sich auf das Melken einer Kuh bezogen, stellt dies keine Beschränkung dar. Bei den zu melkenden Tieren kann es sich um Kühe, Ziegen, Schafe, Dromedare, Stuten oder Yaks und weitere Tierarten handeln.

Weitere Vorteile und Einzelheiten der Erfindung werden anhand des in der Zeichnung dargestellten Ausführungsbeispiels erläutert, ohne dass der Gegenstand der Erfindung auf dieses konkrete Ausführungsbeispiel beschränkt wird. Es zeigen:
- Fig. 1:: schematisch eine Einrichtung zur Ermittlung der Qualität und/oder der Zusammensetzung von Milch,
- Fig. 2:: die Höhe eines Messsignals in Abhängigkeit vom Füllstand und Amaranthkonzentration in der Milch bei einer Auswertung der Lichtstärke des roten Lichts,
- Fig. 3:: ein Diagramm und die Abhängigkeit des Messsignals vom Füllstand und der Amaranthkonzentration in Milch bei der Auswertung des grünen Lichts,
- Fig. 4:: schematisch und perspektivisch eine Vorrichtung,
- Fig. 5:: schematisch und perspektivisch das Gehäuse der Vorrichtung nach Fig. 4,
- Fig. 6.: Schematisch und perspektivisch das Gehäuse der Vorrichtung mit Anschlussstutzen,
- Fig. 7:: schematisch und perspektivisch die Messkammer der Vorrichtung,
- Fig. 8:: die Lage des Sensors in der Vorrichtung,
- Fig. 9:: schematisch und perspektivisch eine zweite Ausführungsform der Vorrichtung,
- Fig. 10:: in einer perspektivischen Ansicht das Gehäuseoberteil der Vorrichtung nach Fig. 9,
- Fig. 11:: im Schnitt das Gehäuseoberteil,
- Fig. 12:: in einer Schnittdarstellung eine Dichtung,
- Fig. 13:: das Gehäuseunterteil in einer perspektivischen Ansicht,
- Fig. 14:: schematisch einen Regelkreis zur dynamischen Kalibrierung des Farbsensors, und
- Fig. 15:: schematisch einen typischen Verlauf des ADC-Signals für einen Farbkanal.

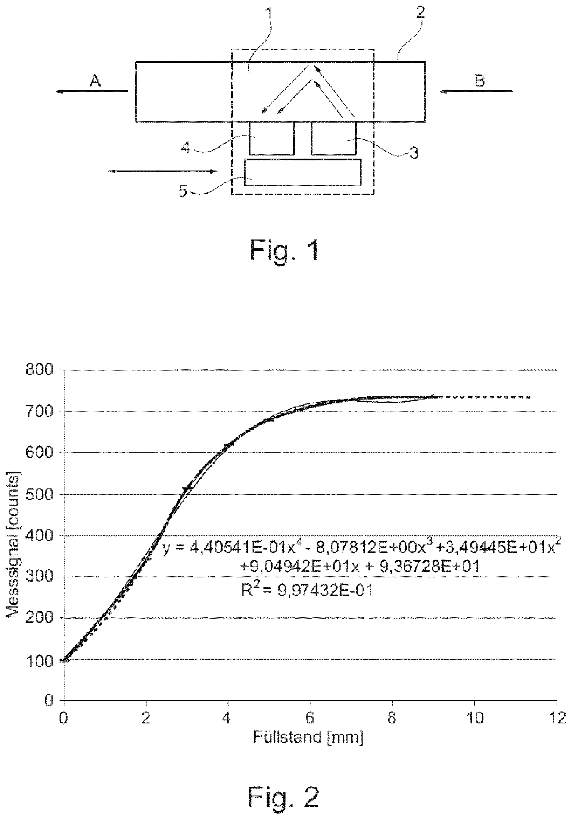

In der Fig. 1 ist schematisch eine Vorrichtung zur Ermittlung der Qualität und/oder der Zusammensetzung von Milch dargestellt. Die Vorrichtung umfasst eine Kammer 1. Die Kammer 1 ist gebildet durch einen Leitungsabschnitt 2. Der Leitungsabschnitt 2 ist vorzugsweise aus Glas. Der Leitungsabschnitt 2 kann auch aus einem anderen Werkstoff gebildet sein, wobei sichergestellt werden muss, dass die Strahlung in die Kammer eintreten und austreten kann. Mit den Pfeilen A und B ist die Flussrichtung einer Milch, die während eines Melkvorgangs gewonnen wird, bezeichnet. Die Milch durchströmt die Kammer 1 in dem Leitungsabschnitt 2. Die Kammer 1 ist vorzugsweise so ausgebildet, dass Lichteinflüsse aus der Umgebung nicht in die Kammer 1 gelangen können. Sollten Lichteinflüsse von der Umgebung vorhanden sein, so können diese durch entsprechende optische Filter und/oder Messung mit ausgeschalteter Lichtquelle kompensiert und/oder berücksichtigt werden.

Mit dem Bezugszeichen 3 ist eine Lichtquelle bezeichnet. Die Lichtquelle emittiert wenigstens eine Strahlung einer vorgegebenen Wellenlänge. Bei der Lichtquelle kann es sich um eine lichtemittierende Diode (light-emitting diode LED) oder eine Laserdiode handeln. Die Lichtquelle kann auch mehrere einzelne Lichtquellen aufweisen, mittels derer die sich in der Kammer befindende Milch mit rotem, grünem und/oder blauem Licht bestrahlt wird. Es ist auch möglich, dass die Lichtquelle weißes Licht abstrahlt und auf der Empfängerseite einzelne Spektralanteile des reflektierten Lichts einzeln bestimmt werden. Hierzu kann wenigstens ein optischer Filter vorgesehen sein.

Das in die Kammer eintretende Licht wird wenigstens teilweise reflektiert. Der Verlauf der Lichtstrahlen ist in Fig. 1 schematisch angedeutet. Ein Empfänger 4 ermittelt periodisch und/oder zeitgleich die von der Milch reflektierten spektralen Farbanteile (rot, grün, blau). Sowohl die Lichtquelle als auch der Empfänger sind mit einer Steuerung 5 verbunden. Mittels der Steuerung 5 kann die Lichtquelle 3 angesteuert werden, insbesondere der Messvorgang ausgelöst werden. Die Steuerung 5 kann mit weiteren Komponenten eines Melksystems verbunden sein. Insbesondere kann die Steuerung bspw. mit einem Bauteil verbunden sein, mittels dessen das Vorhandensein von Milch in der Leitung verifiziert wird. Das Bauteil übermittelt ein Signal an die Steuerung 5, durch welches ein Messvorgang ausgelöst werden kann. Dieses Bauteil kann auch dazu dienen, den Messvorgang oder eine Vielzahl von aufeinanderfolgenden Messvorgängen abzuschließen, wenn keine Milch in der Milchleitung vorhanden ist.

Die Steuerung kann auch ein Signal von einer Reinigungseinheit erhalten, so dass, wie nachstehend ausgeführt wird, auch ein Reinigungsvorgang mittels der Vorrichtung überwacht werden kann.

Vorzugsweise weist die Steuerung einen Speicher auf, in dem die Kennwerte gespeichert sind. Zum Wertepaar Füllstand und Stärke der reflektierten Strahlung wird ein Kennwert ermittelt. Dieser Kennwert kann mit einem Referenzwert verglichen werden, so dass eine Aussage über die Zusammensetzung und/oder Qualität der Milch getroffen werden kann.

Vorzugsweise erfolgt die Messung periodisch, so dass mehrere Messwerte während eines Melkvorgangs erhalten werden können. Die Messung kann auch gleichzeitig erfolgen und zwar in dem Sinne, dass mehrere von der Milch reflektierte spektrale Anteile des Lichts zur Ermittlung des Kennwerts angezogen werden.

Zur Bestimmung des Blutgehalts werden die vom Empfänger erfassten roten und grünen Spektralanteile des Lichts genutzt.

Fig. 2 zeigt den Verlauf der Messwerte des roten Anteils in Abhängigkeit vom Füllstand. Der rote Spektralanteil, den der Empfänger ermittelt, liefert Rückschlüsse über den Füllstand der Milch in der Kammer. Die Darstellung in Fig. 2 zeigt, dass der Füllstand im Wesentlichen unabhängig von einer Konzentration von Amaranth in der Milch ist. Diese Daten können analytisch beschrieben werden, so dass eine mathematische Beziehung zwischen dem Werten des Empfängersignals und dem Füllstand in der Kammer bereitgestellt werden kann. Amaranth ist ein roter Farbstoff, der gut milchlöslich ist und als gutes Substitut für Blut in der Milch bei den Versuchen diente. Es besteht eine unmittelbare Korrelation zwischen einer Amaranth- und Hämoglobinkonzentration in Milch, so dass aus der Kenntnis der Amaranthkonzentration in der Milch eine Aussage über die entsprechende Hämoglobinkonzentration in der Milch und somit auch über Blut in der Milch getroffen werden kann. Es wird daher im Folgenden auf Hämoglobin bezuggenommen.

Aus der Stärke des von der Milch reflektierten roten Lichts wird der Füllstand der Milch in der Kammer 1 bestimmt. Wird die sich in der Kammer 1 befindende Milch mit rotem Licht bestrahlt, so kann aus der reflektierten Lichtmenge der Füllstand der Milch in der Kammer bestimmt werden. Es ist auch möglich, den roten Spektralanteil des reflektierten Lichts auszuwerten, wenn die Lichtquelle breitbandig ist. Aus dem Messergebnis erfolgt die Kenntnis über den Füllstand in der Kammer 1.

Mittels des grünen Spektralanteils und des bekannten Füllstands der Milch in der Kammer kann eine Aussage über die Hämoglobinkonzentration in Milch (µmol/L; µmol pro Liter) ermittelt werden.

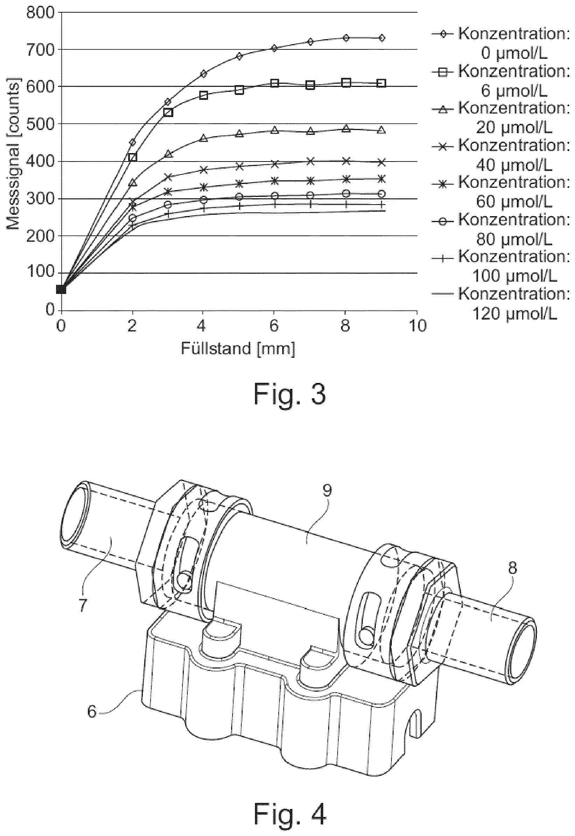

Fig. 3 zeigt den Zusammenhang zwischen dem grünen Spektralanteil, dem Füllstand und der daraus resultierten Hämoglobinkonzentration in der Milch. Aus dem Wertepaar Füllstand und Stärke der reflektierten Strahlung kann ein Kennwert aus gespeicherten Kennwerten ermittelt werden. Die Verläufe, wie sie in der Fig. 3 dargestellt sind, können gespeichert sein. Es besteht auch die Möglichkeit, dass die Kennwerte in Form einer Tabelle, insbesondere einer Look-up-Tabelle gespeichert sind. Alternativ oder zusätzlich kann für die Bestimmung eines Kennwerts der Füllstand und die Stärke der reflektierten Strahlung als Parameter in einer mathematischen Beziehung verwendet werden.

Aus der Darstellung in der Fig. 3 folgt bspw. dass bei einem Füllstand der Kammer von 4 mm und einem Messsignal des Empfängers von ca. 300 counts eine Hämoglobinkonzentration von 80 µmοl/L in der Milch vorliegt.

Die Arbeitsweise der Vorrichtung zur Ermittlung der Qualität und/oder der Zusammensetzung der Milch ist anhand der Messkammer 1 beschrieben worden, welche von der Milch durchströmt wird. Weist die Messkammer ein konstantes, vorgegebenes Messvolumen auf, so bedarf es lediglich der Auswertung des grünen Spektralanteils des Lichts, um zu dem gewünschten Ergebnis zu gelangen.

Die Messung kann als eine Multiplexmessung durchgeführt werden. Die Lichtquelle bzw. Lichtquellen strahlen vorzugsweise monochromes Licht aus. Strahlt bzw. strahlen die Lichtquellen ein breitbandiges Licht aus, so wird der Empfänger mit entsprechenden schmalbandigen Filtern ausgestattet, um die Spektralanteile ermitteln zu können.

Mittels des blauen Spektralanteils des Lichts kann eine Detektion von wässrigen Anteilen in der Milch erreicht werden. Dies ist vorzugsweise dann der Fall, wenn in der Milch Flocken vorhanden sind.
Ist festgestellt worden, dass in der Milch Blut vorhanden ist, so wird vorgeschlagen, dass eine Reinigung des Melksystems zumindest teilweise erfolgt. Die Überwachung der Reinigung erfolgt vorzugsweise mittels des blauen Spektralanteils des reflektierten Lichts. Die Reinigung kann auch dann erfolgen bzw. die Überwachung kann auch dann erfolgen, wenn dies notwendig ist.

Ist festgestellt worden, dass der Kennwert einen vorgegebenen Referenzwert überschreitet, so kann über die Behandlung der Milch entschieden werden. Handelt es sich bei der Milch um nicht verkehrsfähige Milch, so wird diese in einen Tank für nicht verkehrsfähige Milch geleitet, wodurch eine klare Separation zwischen verkehrsfähiger und nicht verkehrsfähiger Milch erreicht wird. Zusätzlich oder alternativ kann auch ein Signal an den Melker gesendet werden, dass die sich beim Melken befindende Kuh eine Problemkuh darstellt.

Die Kalibrierung des Systems kann mit Hilfe eines tierindividuellen Weiß der Milch erfolgen. Alternativ kann ein herdenspezifisches Weiß zur Kalibrierung herangezogen werden. Da die Tiere in Verbindung mit einem bevorstehenden Melkvorgang, insbesondere mittels der bekannten Technik identifiziert werden, können bei einem vorhandenen Herden-Managementsystem, welches mit der Steuerung der erfindungsgemäßen Vorrichtung signaltechnisch verbunden ist, Informationen zur Verfügung stellen, die für die Vorrichtung relevant sein können. Die erfindungsgemäße Vorrichtung kann auch Informationspakete an das Herdenmanagementsystem übermitteln.

Fig. 4 zeigt schematisch und perspektivisch ein Ausführungsbeispiel einer Vorrichtung zur Ermittlung der Qualität und/oder der Zusammensetzung von Milch. Die Vorrichtung weist ein Gehäuse mit einem unteren Gehäuseteil 6 und einem oberen Gehäuseteil 9 auf. Innerhalb des oberen Gehäuseteils 9 ist vorzugsweise ein Glasrohr angeordnet, welches die Kammer 1 bildet. Mit dem oberen Gehäuseteil 9 sind Anschlussstutzen 7, 8 verbunden. Die Anschlussstutzen 7, 8 sind in dem dargestellten Ausführungsbeispiel bajonettartig mit dem oberen Gehäuseteil 9 verbunden. Die Anschlussstutzen 7, 8 sind lösbar.

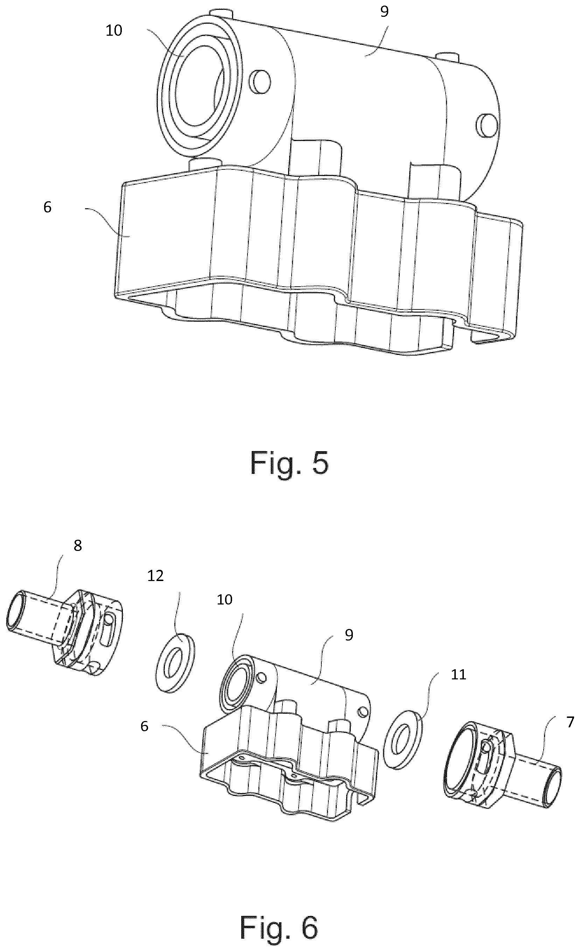

Fig. 5 und 6 zeigen die Vorrichtung mit dem Glasrohr 10, welches in dem oberen Gehäuseteil 9 angeordnet ist. Das obere Gehäuseteil 9 ist so ausgebildet, dass Umgebungslicht nicht in das Glasrohr 10 bzw. in die Kammer gelangen kann. Zur fluiddichten Verbindung der Anschlussstutzen 7, 8 mit dem oberen Gehäuseteil 9 sind Dichtungen 11, 12 vorgesehen. Das Glasrohr 10 ist vorzugsweise mit dem oberen Gehäuseteil 9 verklebt.

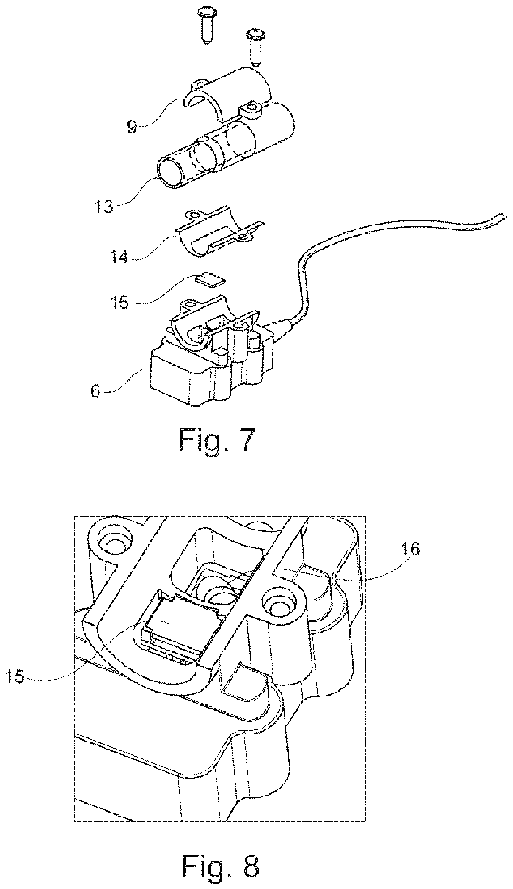

Fig. 7 zeigt ein zweites Ausführungsbeispiel der Vorrichtung. Anstelle eines Glasrohrs ist ein Rohr bzw. Leitungsabschnitt 13 vorgesehen. Der Leitungsabschnitt 13 besteht vorzugsweise aus einem Kunststoff, insbesondere einem lebensmittelverträglichen Kunststoff wie bspw. PSU (Polysulfon). Der Leitungsabschnitt 13 ist mit dem oberen Gehäuseteil 9 verbunden. Im Bereich der Aufnahme des Leitungsabschnitts 13 ist eine Dichtung 14 vorgesehen. Dieser Aufbau der Vorrichtung ermöglicht einen Austausch des Leitungsabschnitts 13. Mit dem Bezugszeichen 15 ist ein optischer Filter bezeichnet.

Der optische Filter 15 ist oberhalb eines Empfängers angeordnet. Die Anordnung des Filters 15 zeigt die Fig. 8. In Fig. 8 ist gleichfalls eine Lichtquelle 16 in Form einer LED dargestellt.

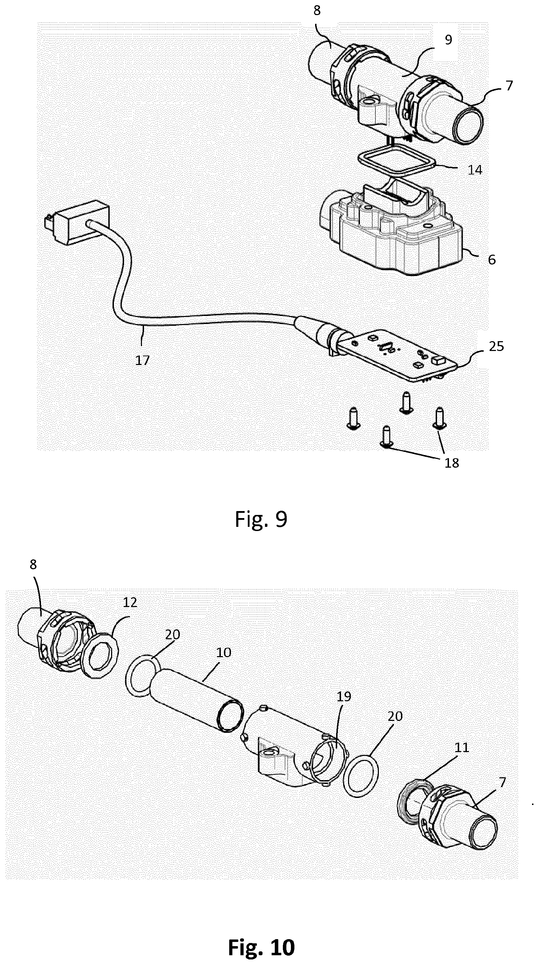

In der Fig. 9 ist perspektivisch ein zweites Ausführungsbeispiel einer Vorrichtung zur Ermittlung der Qualität und/oder der Zusammensetzung von Milch dargestellt. Die Vorrichtung weist ein Gehäuse auf, welches zweiteilig ausgebildet ist. Das Gehäuse hat ein unteres Gehäuseteil 6 und ein oberes Gehäuseteil 9. Das obere Gehäuseteil 9 ist mit dem unteren Gehäuseteil 6 vorzugsweise lösbar verbunden. Zwischen dem unteren Gehäuseteil 6 und dem oberen Gehäuseteil 9 ist eine Dichtung 14 angeordnet. In dem unteren Gehäuseteil 9 ist bspw. eine Elektronikkarte 25 mit den entsprechenden elektronischen Bauteilen mittels Schrauben 18 befestigt. Die Elektronikkarte 25 ist über eine Signalleitung 17 mit einer nicht dargestellten Auswerteeinheit verbunden.

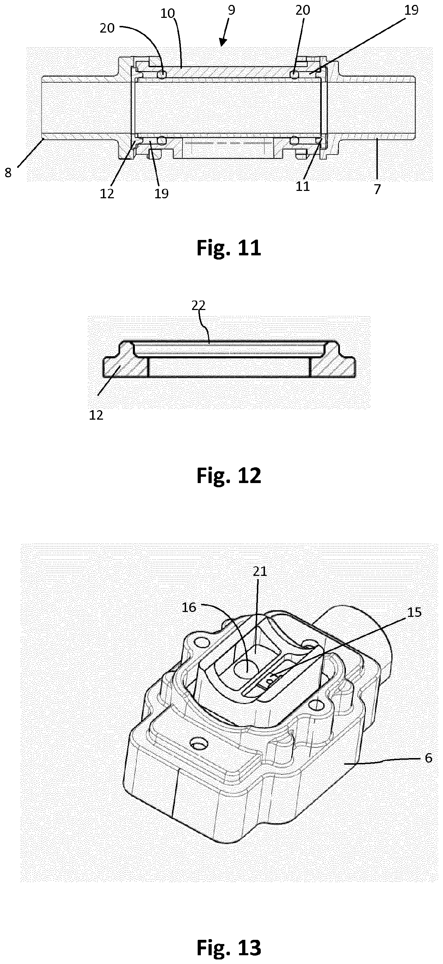

Das obere Gehäuseteil 9 ist in Fig. 10 schematisch und perspektivisch dargestellt. Mit dem oberen Gehäuseteil 9 sind Anschlussstutzen 7, 8 lösbar verbunden. Die Verbindung des oberen Gehäuseteils 9 mit dem Anschlussstutzen 7, 8 ist in dem dargestellten Ausführungsbeispiel bajonettartig ausgebildet. Zwischen den Anschlussstutzen 7, 8 und der Stirnfläche des oberen Gehäuseteils 9 ist jeweils eine Dichtung 11, 12 vorgesehen. Innerhalb des oberen Gehäuseteils 9 ist ein Glasrohr 10 positioniert. In den jeweiligen Endbereichen des oberen Gehäuseteils 9 sind Ausnehmungen 19 vorgesehen, wie dies insbesondere aus der Fig. 11 ersichtlich ist. In den Ausnehmungen 19 ist jeweils eine Dichtung 20 angeordnet. Die Dichtung 20 ist als O-Ring ausgebildet. Die Dichtungen 20 positionieren das Glasrohr 10 innerhalb des oberen Gehäuseteils 9. Ist das Glasrohr 10 innerhalb des oberen Gehäuseteils 9 mittels der Dichtungen 20 angeordnet worden, so wird vorzugsweise der Spalt von der Stirnseite her des oberen Gehäuseteils 9 mit einer Klebe- bzw. Dichtmasse gefüllt. Die Dichtungen 20 stellen sicher, dass die Klebe- bzw. Dichtmasse nicht in den inneren Sensorbereich, das heißt den Bereich zwischen den Dichtungen 20 gelangen kann.

Das Glasrohr 10 ist annähernd so lang wie die axiale Erstreckung des oberen Gehäuseteils 9. Dadurch wird das Glasrohr vor Beschädigungen an seinen Stirnseiten geschützt. Es ist nicht zwingend, dass ein Glasrohr verwendet wird. Ein Glasrohr ist jedoch bevorzugt, da die Komponenten der Milch, insbesondere Wasser, nicht durch das Glasrohr hindurchtreten können.

Aus der Fig. 11 ist ersichtlich, dass zwischen den Anschlussstutzen 7, 8 des oberen Gehäuseteils 9 jeweils eine Dichtung 11, 12 angeordnet ist.

Die Fig. 12 zeigt die Dichtung 12 in einem Abschnitt. Die Dichtung 12 ist mit einer Wulst 22 versehen. Die Wulst 22 greift in den Spalt, der durch das Glasrohr 10 und die Ausnehmung 19 gebildet ist. Durch diese Ausgestaltung der Dichtung 12 wird sichergestellt, dass die Dichtung 12 durch das im Glasrohr herrschende Vakuum nicht in den freien Strömungsquerschnitt gezogen wird. Dadurch kommt es zu keiner Einschnürung des Querschnitts im Übergangsbereich zwischen dem Glasrohr 10 und dem Anschlussstutzen 7 bzw. 8. Die Anschlussstutzen 7, 8 und die Dichtungen 20 sind vorzugsweise so ausgebildet, dass diese lichtdicht sind. Der wenigstens eine Anschlussstutzen kann wenigstens teilweise aus einem Kunststoff, insbesondere aus schwarzen PSU (Polysulfon) gebildet sein.

In der Fig. 13 ist das untere Gehäuseteil 6 dargestellt. In dem unteren Gehäuseteil 6 befindet sich eine Lichtquelle 16 und ein optischer Filter 15 sowie die weiteren elektronischen Komponenten, welche aus der Darstellung nach Fig. 13 nicht ersichtlich sind.

Die sich in dem unteren Gehäuseteil 6 befindenden Bauteile sind vorzugsweise mit einer geeigneten Dichtmasse 21 vergossen, so dass ein funktionierendes Dichtkonzept innerhalb des unteren Gehäuseteils 6 verwirklicht werden kann. Des weiteren wird das Luftvolumen zwischen der Lichtquelle 16 und dem Glasrohr 10 minimiert.

Bei der Verwendung der vorstehend erläuterten Vorrichtungen ist die Vorrichtung vorzugsweise so angeordnet, dass das Glasrohr 10 im Wesentlichen waagerecht positioniert ist. In eingebautem Zustand befindet sich das Glasrohr 10 oberhalb des unteren Gehäuseteils 6.

Vor der Vorrichtung kann eine Beruhigungsstrecke für den Milchfluss vorgesehen sein. Diese Beruhigungsstrecke kann bspw. dadurch verwirklicht werden, dass der Milchschlauch, in dem die Milch zu der Vorrichtung zugeführt wird, im Wesentlichen waagerecht benachbart zu der Vorrichtung verlegt wird.

Eine Kalibrierung der erfindungsgemäßen Vorrichtung erfolgt so, dass zunächst die Kammer 1 bis zu einem vorgegebenen Füllstand mit "weißer" Milch gefüllt wird. Vorzugsweise wird die Kammer 1 vollständig mit weißer Milch gefüllt. Der Strom durch die Lichtquelle (LED) und/oder der Lichtquellen und somit die Lichtstärke wird so eingestellt, dass der Empfänger oder Empfänger für die relevanten Wellenlänge des reflektierten Lichts jeweils ein Messsignal vorgegebener Höhe liefert.

Nachdem der erste Kalibrierungsschritt für den Weißpunkt erfolgte, erfolgt eine Kalibrierung der erfindungsgemäßen Vorrichtung für wenigstens einen Rotpunkt. Hierzu wird die Kammer mit wenigstens einer Milch mit einer vorgegebenen, bekannten Hämoglobinkonzentration oder ersatzweise Amaranthkonzentration gefüllt. Der Füllstand in der Kammer ist vorgegeben. Es erfolgt nun eine Messung und so ermittelten Messwerte dienen zur sensorindividuellen Skalierung der hinterlegen Tabellen (Look-up-Tabellenkennwerte).

Mittels dieser Vorgehensweise wird einerseits die Stärke der wenigstens einen Lichtquelle und die Empfindlichkeit des wenigstens einen Sensors eingestellt und etwaige optische Toleranzen und/oder Einbautoleranzen der erfindungsgemäßen Vorrichtung ausgeglichen.

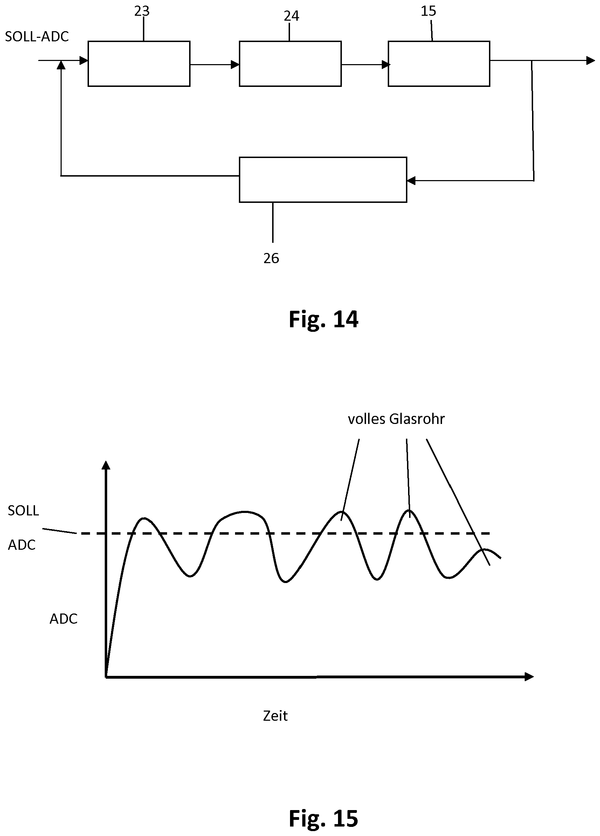

Die Lichtquelle, die innerhalb der Vorrichtung verwendet wird, unterliegt auch Alterungseinflüssen, so dass Milch-Weiß-Unterschiede entstehen können. Die Milch-Weiß-Unterscheide können auch herden- bzw. tierindividuell sein. Ein durch die Vorrichtung gemessene Weiß-ADC-Wert (Weiß-Analog-DigitalUmsetzer-Wert) kann im Betrieb vom Referenz-Weiß bspw. durch eine Alterung der Lichtquelle, die Futtersorte oder den Fettgehalt der Milch beeinflusst werden. Zur dynamischen Kalibrierung wird ein Regelkreis, wie er in der Fig. 14 dargestellt ist, vorgeschlagen. Der Regelkreis weist einen Regler 23, eine Stromquelle 24, eine Lichtquelle 15 des Farbsensors auf. Die Kalibrierung der Vorrichtung erfolgt beim gefüllten Glasrohr 10 mit einer weißen Flüssigkeit. Es erfolgt eine kontinuierliche Erfassung der reflektierten Lichtstärke mittels der Vorrichtung. Ein typischer Signalverlauf ist in der Fig. 16 dargestellt. Der Milchfluss während eines Melkvorgangs ist nicht zwingend periodisch, sondern pulsartig, wie dies aus der Fig. 16 entnehmbar ist. Je voller das Glasrohr 10 gefüllt ist, desto mehr Licht wird von der Vorrichtung erfasst. Entsprechend der Darstellung nach Fig. 16 ist eine periodische Art des Sättigungssignals zu sehen. Diese Sättigung entspricht dem Zeitpunkt, an dem das Glasrohr komplett mit Milch gefüllt ist. Gemittelt wird eine Abweichung vom ADC-Signal zum Soll-ADC Weiß-Signal bestimmt. Der Regler 23 erhält eine Regelabweichung und steuert die Stromquelle 24 der Lichtquelle 16 des Farbsenors so lange, bis sich die Regelabweichung unterhalb einer Minimalabweichung befindet. Das ADC-Signal ist ein digitales Signal, welches aus einem analogen Signal gewonnen wurde. Dies erfolgt mittels eines entsprechenden Wandlers (Analog-Digital-Konverter).

### Bezugszeichenliste

- 1: Kammer
- 2: Leitungsabschnitt
- 3: Lichtquelle
- 4: Empfänger
- 5: Steuerung
- 6: unteres Gehäuseteil
- 7: Anschlussstutzen
- 8: Anschlussstutzen
- 9: oberes Gehäuseteil
- 10: Glasrohr
- 11, 12: Dichtung
- 13: Leitungsabschnitt
- 14: Dichtung
- 15: Filter
- 16: Lichtquelle
- 17: Signalleitung
- 18: Schraube
- 19: Ausnehmung
- 20: Dichtmasse
- 21: Dichtung
- 22: Wulst
- 23: Regler
- 24: Stromquelle
- 25: Elektronikkarte

## Patentansprüche

1. Verfahren zur Ermittlung der Qualität und/oder der Zusammensetzung von Milch während eines Melkvorgangs, bei dem ein Mindestmilchstrom, der durch eine Kammer (1) fliesst, delektiert wird, danach der Füllstand der die Kammer (1) durchströmenden Milch in der Kammer (1) bestimmt wird, die Milch mit wenigstens einer Strahlung einer vorgegebener Wellenlänge bestrahlt wird, die Stärke der reflektierten Strahlung gemessen wird und ein zum Wertepaar Füllstand und Stärke der reflektierten Strahlung zugehöriger Kennwert aus gespeicherten Kennwerten ermittelt wird.

2. Verfahren nach Anspruch 1, bei dem die Milch mit rotem Licht bestrahlt und aus der Stärke des reflektierten roten Lichts der Füllstand der Milch bestimmt wird.

3. Verfahren nach Anspruch 1 oder 2, bei dem die vorgegeben Wellenlänge der Wellenlänge des grünen und/oder blauen Lichts entspricht.

4. Verfahren nach Anspruch 2 oder 3, bei dem die Milch nacheinander oder gleichzeitig mit einem roten, grünen und/oder blauen Licht bestrahlt wird.

5. Verfahren nach einem der Ansprüche 1 bis 4, bei dem die Milch mit pulsierender Strahlung bestrahlt wird.

6. Verfahren nach einem der Ansprüche 1 bis 5, bei dem die Kennwerte in Form einer Tabelle, insbesondere einer Look up Tabelle gespeichert sind.

7. Verfahren nach einem der Ansprüche 1 bis 5, bei dem für die Bestimmung eines Kennwertes der Füllstand und die Stärke der reflektierten Strahlung als Parameter in einer mathematischen Beziehung verwendet werden.

8. Verfahren nach einem der Ansprüche 1 bis 7, bei dem ein Milchstrom, insbesondere ein Mindestmilchstrom detektiert wird und danach der Füllstand bestimmt wird.

9. Verfahren nach einem der Ansprüche 1 bis 8, bei dem die Strahlung eine monochromatische Strahlung ist.

10. Verfahren nach Anspruch 1 und einem der Ansprüche 3 bis 9, bei dem der Füllstand kapazitiv oder induktiv bestimmt wird.

11. Verfahren nach einem der Ansprüche 1 bis 10, bei dem wenigstens ein Sensor mit wenigstens einer Lichtquelle (3) und wenigstens einem Empfänger (4) vorgesehen ist, wobei der Sensor auf ein herdenspezifisches oder tierindividuelles Weiß der Milch kalibriert wird.

12. Verfahren nach Anspruch 1 bis 11 bei dem der Sensor eine rote Licht abstrahlende Lichtquelle (3) und einen rotes Licht empfangenden Empfänger (4) aufweist, wobei dieser Sensor mittels einer vorgegebenen roten Flüssigkeit kalibriert wird.

13. Verfahren nach einem der Ansprüche 1 bis 12, bei dem während eines Melkvorgangs nacheinander mehrere Kennwerte ermittelt werden.

14. Verfahren nach einem der Ansprüche 1 bis 13, bei dem in Abhängigkeit von wenigstens einem Kennwert die ermolkene Milch in einen Tank für verwertbare oder nichtverwertbare Milch geleitet wird.

## Claims

1. A method for ascertaining the quality and/or the composition of milk, during a milking operation, in which a minimum milk stream which flows through a chamber (1) is detected, thereafter the fill level of the milk flowing through the chamber (1) is determined in the chamber (1), the milk is irradiated using at least one radiation of a predefined wavelength, the intensity of the reflected radiation is measured, and a characteristic value associated with the value pair fill level and intensity of the reflected radiation is ascertained from stored characteristic values.

2. The method as claimed in claim 1, wherein the milk is irradiated using red light and the fill level (F) of the milk is determined from the intensity of the reflected red light.

3. The method as claimed in claim 1 or 2, wherein the predefined wavelength corresponds to the wavelength of the green and/or blue light.

4. The method as claimed in claim 2 or 3, wherein the milk is successively or simultaneously irradiated using a red, green, and/or blue light.

5. The method as claimed in any one of claims 1 to 4, wherein the milk is irradiated using pulsing radiation.

6. The method as claimed in any one of claims 1 to 5, wherein the characteristic values are stored in the form of a table, in particular a lookup table.

7. The method as claimed in any one of claims 1 to 5, wherein the fill level and the intensity of the reflected radiation are used as parameters in a mathematical relationship for the determination of a characteristic value.

8. The method as claimed in any one of claims 1 to 7, wherein a milk stream, in particular a minimum milk stream, is detected and the fill level is determined thereafter.

9. The method as claimed in any one of claims 1 to 8, wherein the radiation is a monochromatic radiation.

10. The method as claimed in claim 1 and any one of claims 3 to 9, wherein the fill level is determined capacitively or inductively.

11. The method as claimed in any one of claims 1 to 10, wherein at least one sensor having at least one light source (3) and at least one receiver (4) is provided, wherein the sensor is calibrated to a herd-specific or animal-individual white of the milk.

12. The method as claimed in claim 1 to 11, wherein the sensor has a light source (3) which emits red light and a receiver (4) which receives red light, wherein this sensor is calibrated by means of a predefined red liquid.

13. The method as claimed in any one of claims 1 to 12, wherein multiple characteristic values are ascertained successively during a milking operation.

14. The method as claimed in any one of claims 1 to 13, wherein the milked milk is conducted into a tank for milk which can be used or milk which cannot be used in dependence on at least one characteristic value.

## Revendications

1. Procédé permettant de déterminer la qualité et/ou la composition du lait pendant un processus de traite, dans lequel un débit de lait minimum s'écoulant à travers une chambre (1) est détecté et le niveau de remplissage de la chambre (1) par le lait la traversant est déterminé dans le chambre (1), le lait est exposé à au moins un rayonnement ayant une longueur d'onde prédéterminée, l'intensité du rayonnement réfléchi est mesurée et une valeur caractéristique associée à une paire de valeurs constituée par le niveau de remplissage et l'intensité du rayonnement réfléchi est déterminée à partir de valeurs caractéristiques mémorisées.

2. Procédé selon la revendication 1, dans lequel le lait est exposé à de la lumière rouge et le niveau de remplissage par le lait est déterminé à partir de l'intensité de la lumière rouge réfléchie.

3. Procédé selon la revendication 1 ou 2, dans lequel la longueur d'onde prédéterminée correspond à la longueur d'onde d'une lumière verte et/ou bleue.

4. Procédé selon la revendication 2 ou 3, dans lequel le lait est exposé consécutivement ou simultanément à une lumière rouge, verte et/ou bleue.

5. Procédé selon l'une des revendications 1 à 4, dans lequel le lait est exposé à un rayonnement pulsé.

6. Procédé selon l'une des revendications 1 à 5, dans lequel les paramètres sont mémorisés sous la forme d'une table, en particulier d'une table de consultation.

7. Procédé selon l'une des revendications 1 à 5, dans lequel le niveau de remplissage et l'intensité du rayonnement réfléchi sont utilisés en tant que paramètres dans une relation mathématique pour déterminer une valeur caractéristique.

8. Procédé selon l'une des revendications 1 à 7, dans lequel un débit de lait, en particulier un débit de lait minimum, est détecté et le niveau de remplissage est ensuite déterminé.

9. Procédé selon l'une des revendications 1 à 8, dans lequel le rayonnement est un rayonnement monochromatique.

10. Procédé selon la revendication 1 et l'une des revendications 3 à 9, dans lequel le niveau de remplissage est déterminé de manière capacitive ou inductive.

11. Procédé selon l'une des revendications 1 à 10, dans lequel il est prévu au moins un capteur comportant au moins une source de lumière (3) et au moins un récepteur (4), dans lequel le capteur est étalonné par rapport à un niveau de blanc du lait propre au troupeau ou individuel de l'animal.

12. Procédé selon les revendications 1 à 11, dans lequel le capteur comporte une source de lumière (3) rayonnant de la lumière rouge et un récepteur (4) recevant de la lumière rouge, dans lequel ledit capteur est étalonné au moyen d'un liquide rouge prédéterminé.

13. Procédé selon l'une des revendications 1 à 12, dans lequel plusieurs valeurs caractéristiques sont déterminées consécutivement pendant un processus de traite.

14. Procédé selon l'une des revendications 1 à 13, dans lequel le lait de traite est introduit dans une cuve destinée à du lait exploitable ou non exploitable.
